# EUROPEAN PATENT APPLICATION

(11) **EP 4 265 263 A1**
(43) Date of publication of application: **25.10.2023**
(21) Application number: 22382361.8
(22) Date of filing: 19.04.2022
(51) Int. Cl.: A61K 35/76, A61P 31/04, C12N 7/00

(54) **BACTERIOPHAGE SUITABLE FOR TREATING A BACTERIAL INFECTION CAUSED BY PSEUDOMONAS AERUGINOSA**

(71) Applicant: Fundacion Instituto De Investigacion Sanitaria Fundacion Jimenez Diaz, 28040 Madrid (ES); Universidad Complutense De Madrid, 28040 Madrid (ES)
(72) Inventor: GARCÍA QUINTANILLA, Meritxell de Jesús, 28040 Madrid (ES); ESTEBAN MORENO, Jaime, 28040 Madrid (ES); GADEA GIRONÉS, Ignacio, 28040 Madrid (ES); FERNÁNDEZ ROBLAS, Ricardo, 28040 Madrid (ES); AGUILERA CORREA, John Jairo, 28040 Madrid (ES); SANTAMARÍA CORRAL, Guillermo, 28040 Madrid (ES)
(74) Representative: Hoffmann Eitle

(57) **Abstract**

The present invention refers to a bacteriophage deposited at the German Collection of Microorganisms and Cell Cultures GmbH (DSMZ) with deposit number DSM 34223. In a preferred embodiment, the bacteriophage is used for treating bacterial infections caused by *Pseudomonas aeruginosa.*

## Description

### FIELD OF THE INVENTION

The present invention refers to the medial field. Particularly, the present invention refers to a bacteriophage deposited at the German Collection of Microorganisms and Cell Cultures GmbH (DSMZ) with deposit number DSM 34223 on 17^{th} of March 2022. In a preferred embodiment, the bacteriophage is used for treating bacterial infections caused by *Pseudomonas aeruginosa.*

### STATE OF THE ART

*P. aeruginosa* is a prevalent Gram-negative bacillus found in water, oil, different surfaces, medical devices which can be isolated from plants, animals, and humans. Currently, *P. aeruginosa* constitutes one of the main opportunistic pathogens which can cause a wide variety of nosocomial, acute, and chronic infections (including pneumonia, septicemia, urinary tract and surgery site infections), especially in immunocompromised individuals. Cystic fibrosis (CF) patients are particularly vulnerable to *P. aeruginosa* infections being one of the major causes of mortality and morbidity. This pathogen causes chronic lung infection, deterioration of pulmonary function and, in the worst cases, death, and colonizes 30% of children and even 80% of older 25-year-old adults suffering CF.

This bacterium is capable of invading host cells and avoiding host defenses due to an arsenal of virulent secreted factors, such as proteases, elastases, pyocyanins, exotoxin A, phospholipases, exoenzymes, and cell-associated factors (lipopolysaccharides, flagella and pili). Moreover, *P*. *aeruginosa* contains a low permeable outer membrane and multiple transport systems, providing an innate resistance to many antibiotics. In addition to its innate resistance, *P. aeruginosa* is able to develop resistance to almost all available antimicrobials showing resistance against several antimicrobials including fluoroquinolones, β-lactams, and aminoglycosides, with multi-drug resistant (MDR) and extensively-drug resistant (XDR) varieties. The main mechanisms conferring resistance in MDR *P. aeruginosa* consist of alterations in porin channels, efflux pumps, target modifications and β-lactamases (for instance, AmpC and carbapenemases). Antimicrobial resistance can be acquired by selection of mutations in chromosomal genes or by horizontal uptake of resistance determinants. Furthermore, failure of antimicrobial treatments has also been associated with the formation of biofilms.

Owing to the absence of appropriate and effective treatments, researchers are looking for new ways of inhibiting MDR and XDR *P. aeruginosa* strains, being phage therapy one of the most promising methods. Phages against *P. aeruginosa* have been isolated from hospital sewage, seawater, ponds, rivers and wastewater-treatment plants.

However, there is an unmet clinical need of finding reliable bacteriophage candidates able to inhibit the growth of clinical strains of *P. aeruginosa* (including MDR and XDR strains), both in liquid and in mature biofilms. The present invention is thus focused on solving this problem and new strategy is herein provided.

### DESCRIPTION OF THE INVENTION

### Brief description of the invention

The present invention refers to a bacteriophage deposited at the German Collection of Microorganisms and Cell Cultures GmbH (DSMZ) with deposit number DSM 34223 on 17^{th} of March 2022 (hereinafter *"bacteriophage of the invention or FIPA*")*.* In a preferred embodiment, the bacteriophage of the invention is used for treating bacterial infections caused by *P. aeruginosa.*

It is of utmost importance to consider that, according the results provided in the present invention (see **Example** 2 of the present invention), the bacteriophage of the invention is able to inhibit the growth of clinical strains of *P. aeruginosa* (including MDR and XDR strains), both in liquid and in mature biofilms, preferably after 6 and 24 hours of treatment with the bacteriophage.

So, the first embodiment of the present invention refers to a bacteriophage deposited at the German Collection of Microorganisms and Cell Cultures GmbH (DSMZ) with deposit number DSM 34223 on 17^{th} of March 2022.

In a preferred embodiment, the bacteriophage of the invention was deposited at the German Collection of Microorganisms and Cell Cultures GmbH (DSMZ) with deposit number DSM 34223.

The second embodiment of the invention refers to the bacteriophage of the invention for use as a medicament, preferably for use in the prevention or treatment of a bacterial infection caused by *P. aeruginosa.*

The third embodiment of the invention refers to a pharmaceutical composition comprising the bacteriophage of the invention and, optionally, pharmaceutically acceptable excipients or carriers.

The fourth embodiment of the present invention refers to the pharmaceutical composition comprising the bacteriophage of the invention for use as a medicament, preferably for use in the prevention or treatment of a bacterial infection caused by *P. aeruginosa.*

Alternatively, the present invention refers to a method for preventing or treating a bacterial infection caused by *P. aeruginosa* which comprises the administration of a therapeutically effective dose or amount of the bacteriophage of the invention or a pharmaceutical composition comprising the bacteriophage of the invention.

In a preferred embodiment the bacteriophage of the invention is administered along with one or more antibiotics, wherein the antibiotics are administered before, after or the same time than the bacteriophage of the invention. The antibiotics to be administered in combination with the bacteriophage of the invention are preferably selected from: Antipseudomonal beta-lactam antibiotics such as penicillin or cephalosporin, aminoglycoside, carbapenems such as imipenem or meropenem, antipseudomonal quinolones, or combinations thereof.

The last embodiment of the present invention refers to a method of detecting the presence of *P. aeruginosa* in an *in vitro* sample, for instance obtained from a subject, wherein the subject is preferably a patient, comprising contacting said sample with the bacteriophage of the invention and determining whether said bacteriophage kills bacteria in said sample.

For the purpose of the present invention the following terms are defined:
- The term "comprising" is meant including, but not limited to, whatever follows the word "comprising". Thus, use of the term "comprising" indicates that the listed elements are required or mandatory, but that other elements are optional and may or may not be present.
- By "consisting of" is meant including, and limited to, whatever follows the phrase "consisting of'. Thus, the phrase "consisting of" indicates that the listed elements are required or mandatory, and that no other elements may be present.
- By "therapeutically effective dose or amount" is intended an amount that, when administered as described herein, brings about a positive therapeutic response in the patient. The exact amount required will vary from subject to subject, depending on (non-exhaustive list): the species, age, general condition of the subject, the severity of the condition being treated or the mode of administration. An appropriate "effective" amount in any individual case may be determined by one of ordinary skill in the art using routine experimentation, based upon the information provided herein.

### Description of the figures

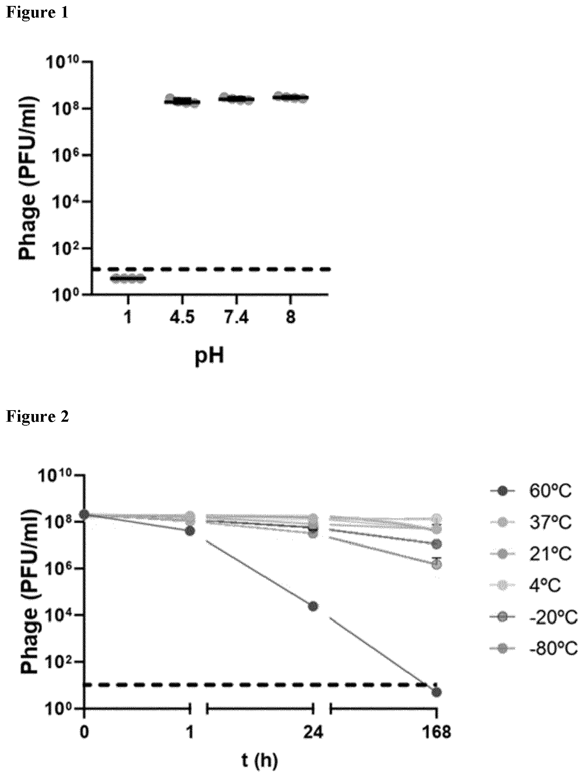

**Figure 1****.** F1PA pH stability. Incubation for 1 h at pH of 1, 4.5, 7.4, and 8. The initial concentration of F1PA was 1.35·10⁸ PFU/ml. It is represented the median and the interquartile range. The segmented line denotes the detection limit of quantification (10 PFU/ml).
**Figure 2****.** F1PA temperature stability. Incubation for 1 h, 24 h and 168 h at temperatures of -80°C, -20°C, 4°C, 21°C, 37°C and 60°C. The bars represent the median and the interquartile range. The segmented line denotes the detection limit of quantification (10 PFU/ml).

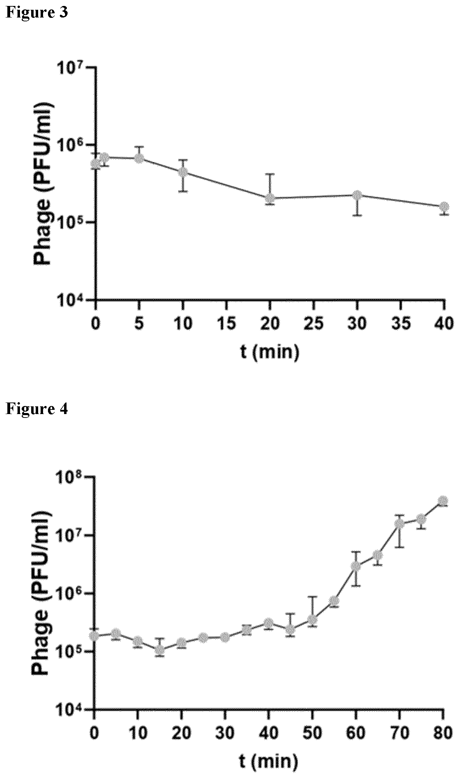

**Figure 3****.** Adsorption of F1PA to host bacterial surface. Plaque forming units per ml (PFU/ml) was measured at 0 min, 1 min, 5 min, 10 min, and every 10 min thereafter for 40 min infecting PAO1 reference strain. The bars represent the median and the interquartile range.
**Figure 4****.** One-step growth curve of F1PA phage is shown. The bars represent the median and the interquartile range.

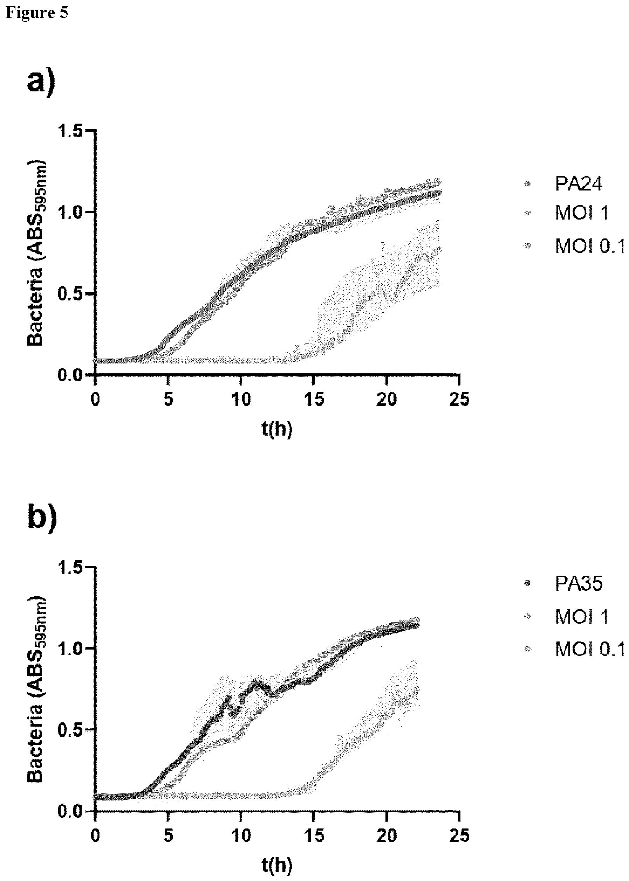

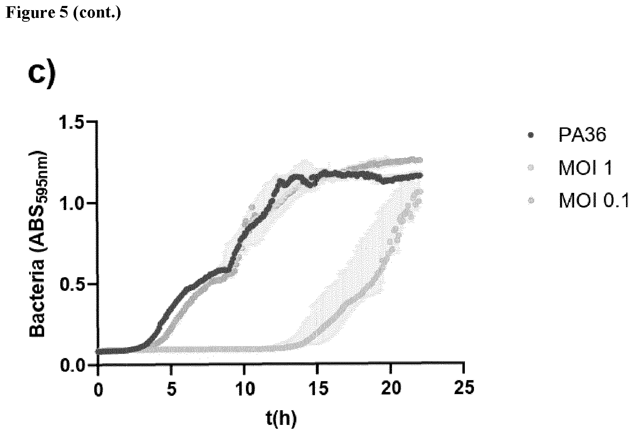

**Figure 5****.** Inhibitory activity of F1PA against clinical *P. aeruginosa* isolates in liquid medium. Three clinical isolates (PA24, PA35 and PA36) were grown in LB medium in absence or presence of F1PA bacteriophage at multiplicity of infection (MOI) of 0.1 and 1 **(a-c).** The grey bars represent the interquartile range.

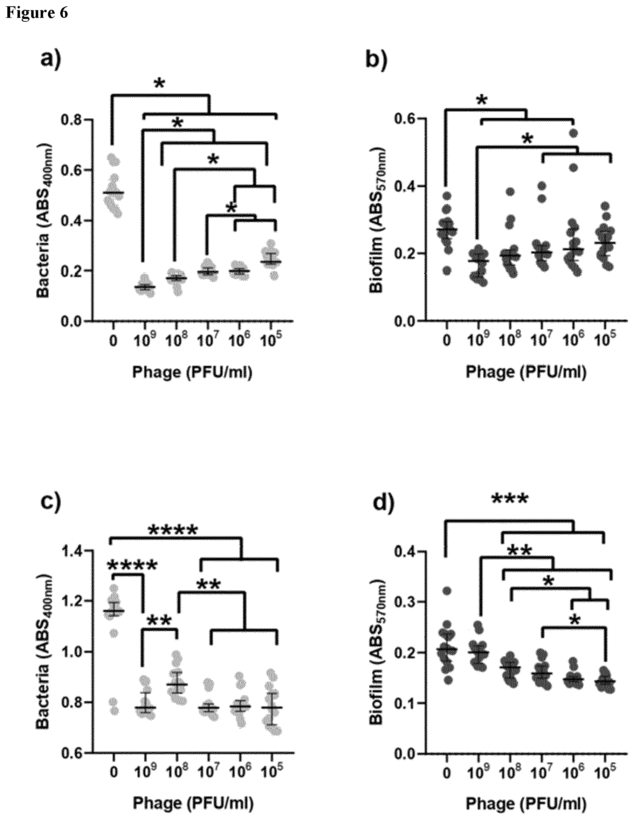

**Figure 6****.** F1PA effect against PAO1 planktonic **(a, c)** and biofilm **(b, d)** forms derived from a biofilm grown for 24h hours and treated with phages during 6h **(a-b)** and 24h **(c-d).** The bars represent the median and the interquartile range. ^{∗}: p-value<0.05, ^{∗∗}: p-value<0.01, ^{∗∗∗}: p-value<0.001, ^{∗∗∗∗}: p-value<0.0001 for Dunn's test pairwise test with a Benjamini-Hochberg's procedure.

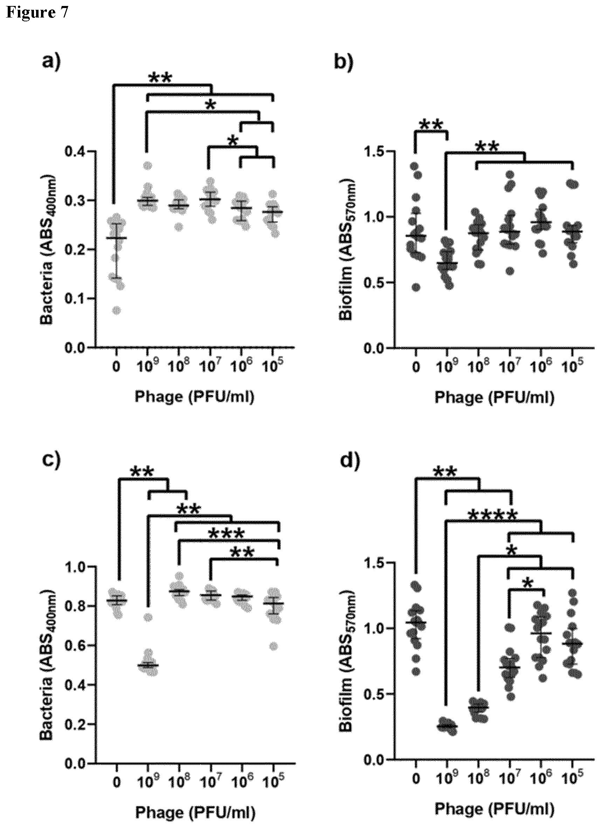

**Figure 7****.** F1PA effect on PA24 clinical strain planktonic **(a, c)** and biofilm **(b, d)** forms derived from a biofilm grown for 24h hours and treated with phages during 6h **(a-b)** and 24h **(c-d).** The bars represent the median and the interquartile range. ^{∗}: p-value<0.05, ^{∗∗}: p-value<0.01, ^{∗∗∗}: p-value<0.001, ^{∗∗∗∗}: p-value<0.0001 for Dunn's test pairwise test with a Benjamini-Hochberg's procedure.

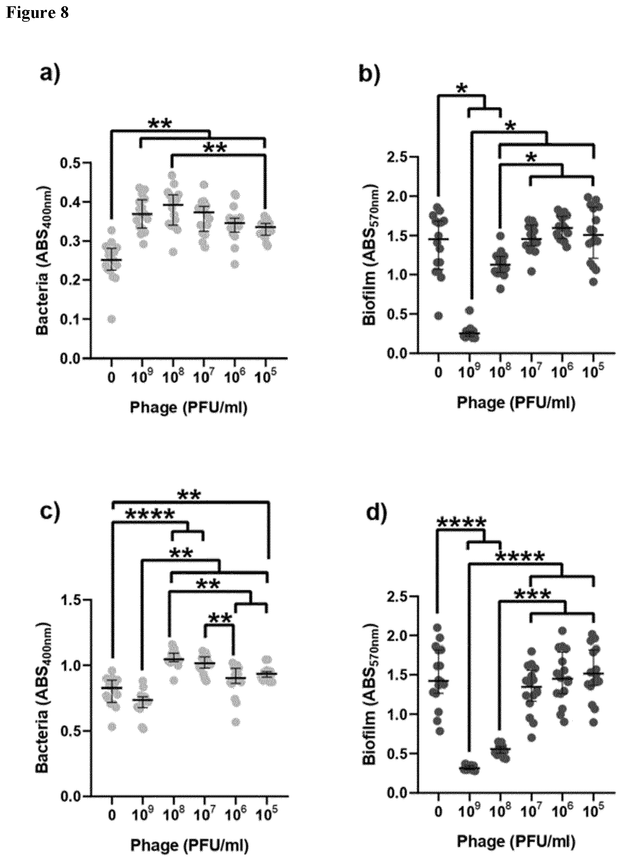

**Figure 8****.** F1PA effect on PA35 clinical strain planktonic **(a, c)** and biofilm **(b, d)** forms derived from a biofilm grown for 24h hours and treated with phages during 6h **(a-b)** and 24h **(c-d).** The bars represent the median and the interquartile range. ^{∗}: p-value<0.05, ^{∗∗}: p-value<0.01, ^{∗∗∗}: p-value<0.001, ^{∗∗∗∗}: p-value<0.0001 for Dunn's test pairwise test with a Benjamini-Hochberg's procedure.

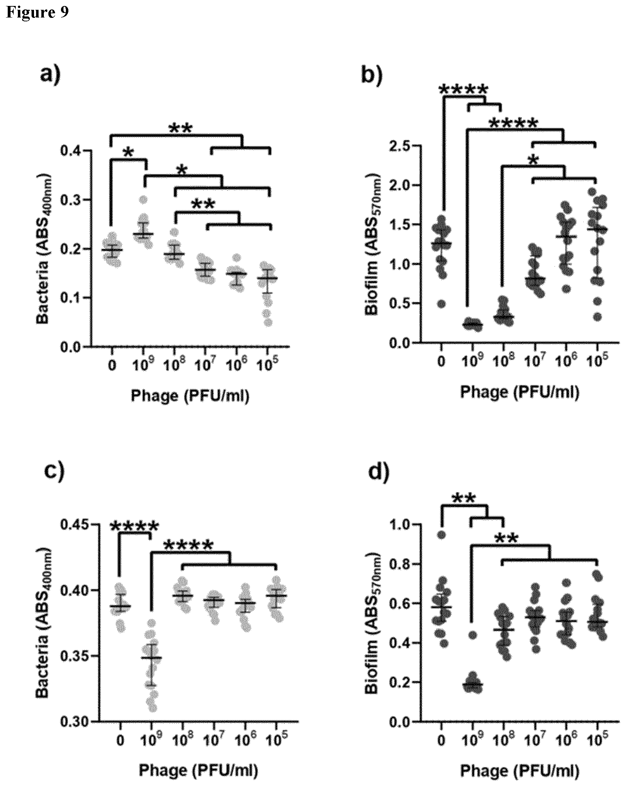

**Figure 9****.** F1PA effect on PA36 clinical strain planktonic **(a, c)** and biofilm **(b, d)** forms derived from a biofilm grown for 24h hours and treated with phages during 6h **(a-b)** and 24h **(c-d).** The bars represent the median and the interquartile range. ^{∗}: p-value<0.05, ^{∗∗}: p-value<0.01, ^{∗∗∗}: p-value<0.001, ^{∗∗∗∗}: p-value<0.0001 for Dunn's test pairwise test with a Benjamini-Hochberg's procedure.

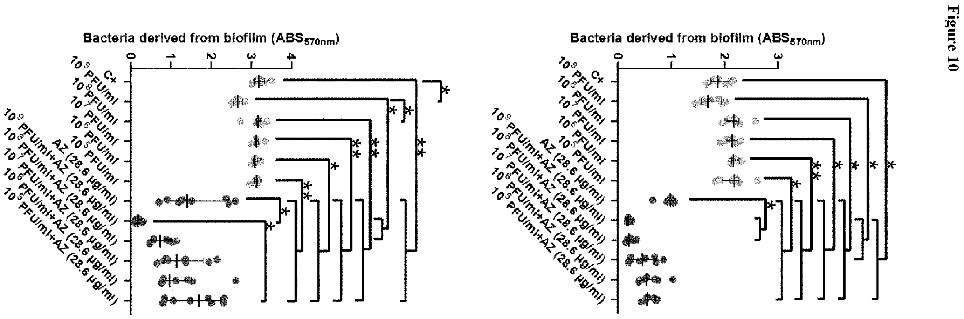

**Figure 10****.** F1PA combined with aztreonam (AZ) (28.6 µg/ml) effect on PAO1, and PA24 clinical strain biofilm growth in planktonic state at 24h **(a-b).** It is represented the median and the interquartile range. ^{∗}: p-value<0.05, ^{∗∗}: p-value<0.01, ^{∗∗∗}: p-value<0.001, ^{∗∗∗∗}: p-value<0.0001 for Dunn's test pairwise test with a Benjamini-Hochberg's procedure.

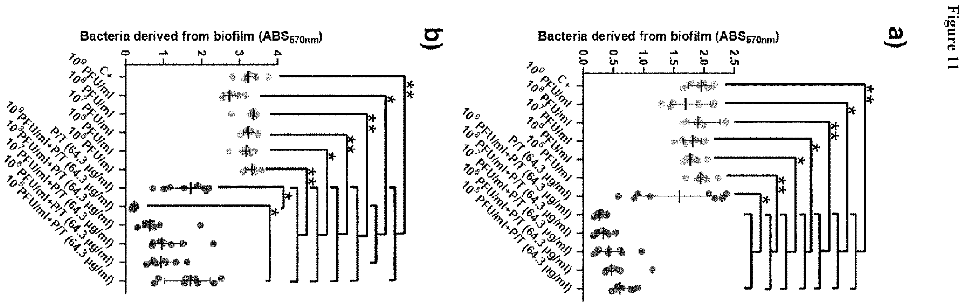

**Figure 11****.** F1PA combined with piperacillin-tazobactam (P/T) (64.3 µg/ml) effect on PAO1, and PA24 clinical strain biofilm growth in planktonic state at 24h **(a-b).** It is represented the median and the interquartile range. ^{∗}: p-value<0.05, ^{∗∗}: p-value<0.01, ^{∗∗∗}: p-value<0.001, ^{∗∗∗∗}: p-value<0.0001 for Dunn's test pairwise test with a Benjamini-Hochberg's procedure

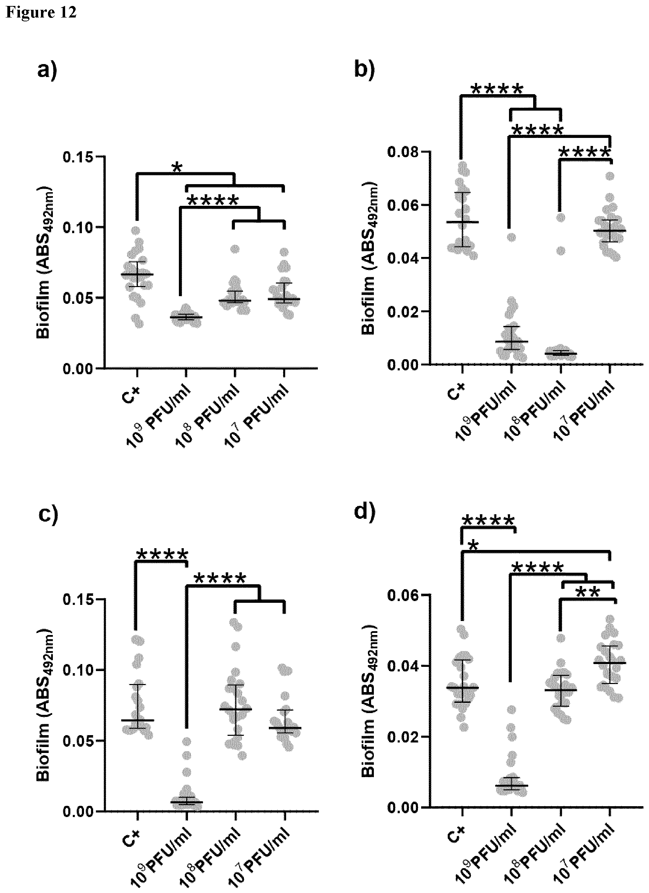

**Figure 12****.** F1PA effect on PAO1, PA24. PA35, and PA36 clinical strain biofilm formation after 24h **(a-d).** It is represented the median and the interquartile range. ^{∗}: p-value<0.05, ^{∗∗}: p-value<0.01, ^{∗∗∗}: p-value<0.001, ^{∗∗∗∗}: p-value<0.0001 for Dunn's test pairwise test with a Benjamini-Hochberg's procedure.

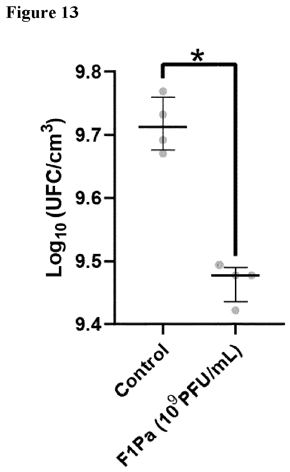

**Figure 13****.** F1PA effect on PAO1 strain biofilm formation developed in wound-like medium (WLM) at 24h. It is represented the median and the interquartile range. ^{∗}: p-value<0.05, ^{∗∗}: p-value<0.01, ^{∗∗∗}: p-value<0.001, ^{∗∗∗∗}: p-value<0.0001 for Mann-Whitney's test.

### Detailed description of the invention

The present invention is illustrated by means of the Examples set below without the intention of limiting its scope of protection.

### Example 1. Material and Methods

### Example 1.1. Bacterial strains and growth conditions

*P. aeruginosa* reference strain ATCC15692 (PA01) was obtained from the American Type Culture Collection (Manassas, VA, USA). *P. aeruginosa* clinical isolates of patients (n=35) were donated by the Department of Microbiology, Hospital Universitario Fundación Jiménez Diaz (HUFJD). *P*. *aeruginosa* clinical isolates were confirmed using MALDI-TOF (Bruker, VIC, Australia). The *P*. *aeruginosa* clinical isolates and PAO1 were stored in Difco^{™} skimmed milk (East Rutherford, NJ, United States) at -80 °C. The clinical isolates were plated from frozen skimmed milk stocks onto tryptic soy agar with 5% sheep blood (TSS) plates (BioMerieux, France) and broth cultures were grown in tryptic soy broth (TSB) (MERCK). Agar plates and broth cultures were incubated at 37 °C with 5% extra CO₂.

### Example 1.2. Bacteriophage Isolation

The *P. aeruginosa* phage (F1PA) was obtained from HUFJD sewage. Samples of 50 ml were centrifuged at 4500 g for 10 min to remove cellular debris and faecal matter. The supernatant was filtered using a 0.22 µm filter to remove bacteria and debris. An amount of 100 µl of filtered solution was combined with 100 µl of the PA01 overnight culture and 3 ml of molten 0.2 % (W/V) LB Agar (LBA) and plated on 1.5 % (W/V) LBA plates via the double-layer agar method. Following overnight incubation, the formation of plaques confirmed the presence of phage. An individual plaque was picked using pipette tips and placed into a 1.5 ml microcentrifuge tube containing 1 ml of sodium magnesium buffer (SM; 100 mM sodium chloride (Panreac Química, Illinois Tool Works, Glenview, Illinois United States); 10 mM magnesium sulfate (Thermo Fisher Scientific, Waltham, MA, USA); 10 mM calcium chloride (Thermo Fisher Scientific, Waltham, MA, USA); 50 mM Tris HCL (Sigma-Aldrich, Castle Hill, NSW, Australia), pH 7.5), and was vortexed vigorously for 5 min and centrifuged at 4000 g for 5 min before 4°C storage.

### Example 1.3. Bacteriophage propagation

A two-step propagation was applied to amplify and purify the isolated phage. For small-scale amplification of phage, 100 µl of PA01 overnight culture was added into 10 mL of TSB. An amount of 100 µl of phage in SM buffer, 150 µl of MgSO₄ 200 mM and CaCl₂ 200 mM was added into the coculture and incubated overnight at 37°C with shaking at 200 rpm. The supernatant containing phage was harvested after centrifugation (4500 rpm, 10 min) and filtration (0.22 µm syringe filter). Phage titration was performed. The small-scale amplification was applied.

For large-scale amplification of phage, 500 µl of the PA01 overnight culture was incubated with 50 ml of TSB for 20 min. After incubation, 100 µl of phage and 2.5 ml of MgSO₄ and CaCl₂ were then added into the coculture and incubated overnight at 37°C with 200 rpm shaking. The phage lysis was harvested as described above. The large-scale amplification was applied.

### Example 1.4. Bacteriophage titration

The phage titer was determined via a double-layer agar method. Briefly, 100 µl of the PA01 overnight broth culture was added to 3 ml of molten 0.2% (W/V) LB agarose (LBA) and overlaid onto a 1.5% (W/V) LBA plate. Phages were serially diluted in SM buffer. The plates were incubated at 37 °C overnight. The phage titer was counted and calculated.

### Example 1.5. Bacteriophage Stability testing

The stability of F1PA phage was tested against a wide pH (1-8) and temperature (-80°C to 60°C) range using a working stock in TSB broth with an initial phage titer of around 2 × 10¹⁰ PFU/mL. Briefly, 10 µl working stock of each phage was suspended in 1 ml SM buffer previously adjusted with 1M NaOH or HCl (Sigma-Aldrich, Castle Hill, NSW, Australia) to yield a pH range of 1-8. The samples were incubated at room temperature for 1 h. The pH stability testing was studied. For thermal stability, 10 µl of working stock phage was suspended in 1 ml SM buffer and incubated at -80°C, -20°C, 4°C, 21°C, 37°C, and 60°C for 1, 24 and 168 h. The phage stability for each experiment was determined by measuring the phage titration. The thermal stability testing was studied.

### Example 1.6. Bacteriophage Adsorption Assays

F1PA was selected for planktonic testing against PA01 reference strain. A volume of 400 µl with 2 McFarland inoculation standards of PAO1 (10⁹ UFC/ml) was diluted 1:100 in fresh TSB broth in a total volume of 40 ml. Phages were added at MOI 0.1. The bacterial suspension was incubated at 37 °C with 180 rpm shaking. After taking an initial 3 ml sample at 0 min, 3 ml samples were taken at 1 min, 5 min, 10 min and every 10 min thereafter for 40 min. The samples were centrifuged at 4000 rpm for 10 min. The supernatant containing the unadsorbed phages was filtered through a 0.22 µm filter and plated using the double-layer agar method. The phage adsorption testing was studied.

### Example 1.7. Bacteriophage one-step growth experiment

In 1 ml of TSB broth, a PAO1 bacterial suspension with a final concentration of 10⁹ CFU/ml and a F1PA filtrate with a final concentration of 10⁷ PFU/mL were mixed. The mixture was incubated at 37°C for 8 min and then centrifuged for 10 min at 5000 rpm. The supernatant was removed, the pellet resuspended in 100 ml of TSB broth and the final suspension was incubated at 37°C with 180 rpm shaking. Aliquots of 0.5 ml of the resulting suspension were taken every 5 min for 80 min and the bacteriophage titer was assessed using double-layer agar method. The latency period was defined as the interval between adsorption of the phage to the host cell and release of phage progeny. The burst size of the phage was expressed as the ratio of the final count of released phage particles between the number of infected bacterial cells during the latency period.

### Example 1.8. Bacteriophage Host Range Analysis

The ability of F1PA to lyse *Pseudomonas aeruginosa* clinical strains obtained from Hospital Universitario Fundación Jiménez Diaz (HUFJD) using spot testing. The host range test was applied. Briefly, 3 µlof the purified phage suspension (10¹⁰ PFU/ml) was spotted onto the surface of the double-layer agar plate previously inoculated with the tested clinical strains. After the absorption of the phage suspension, the plates were incubated overnight at 37°C. Host range was determined by plaque formation. Three replicates were tested for each bacterial strain.

### Example 1.9. Bacteriophage Inhibition assays

The infectivity profile of the F1PA bacteriophage was assessed at multiplicity of infection (MOI) of 0.1, 1 and 10 using inhibition assays in liquid. In brief, the McFarland inoculation standards (0.5±0.02) of each clinical isolate (10⁸ UFC/ml) were prepared and the required volume of phage stock solution (~2 × 10¹⁰ PFU/ml) was added to the corresponding suspension to achieve an MOI of 0.1, 1 and 10. The samples were incubated at 37°C with shaking orbital amplitude of 5 mm. Every 5 min for 22 hours, the OD₅₉₅ value was measured.

### Example 1.10. F1PA effect on pseudomonal biofilm

The F1PA effect on pseudomonal biofilm was determined. Briefly, biofilm formation on the bottom of a MicroWell 96-well flat-bottom plate (Thermo Fisher Scientific, Waltham, MA, USA) was induced by inoculating 100 µl of MHB containing 10⁶ CFU/ml of bacteria per well and the plate was incubated at 37°C and 5% CO₂ for at least 18 h ⁴. After incubation, the supernatant was discarded, 200 µl per well with different concentrations of F1PA in MHB supplemented with 10 mM of Ca²⁺ and 10 mM of Mg²⁺ were deposited and the plate was incubated at 37°C and 5% CO₂ for at least 20 h. After incubation, pseudomonal concentration was also determined by measuring the absorbance at 400 nm and the amount of biofilm was determined by using 3-(4,5-dimethylthiazol-2-yl)-2,5- diphenyltetrazolium bromide (MTT) (Sigma Aldrich, Merck, Darmstadt, Germany), and measuring the absorbance at 570 nm. After administering phage treatment, MBIC was measured at 6 h and 24 h.

### Example 1.11. Inhibition of biofilm formation by bacteriophage

The quantification of inhibition of biofilm by bacteriophage was performed in microtiter plates described by Stepanovic et al. (2007), with modifications. Briefly, biofilm formation on the bottom of a MicroWell 96-well flat-bottom plate (Thermo Fisher Scientific, Waltham, MA, USA) was induced by inoculating 100 µl of TSB supplemented with 1% glucose, 10 mM of Ca²⁺ and 10 mM of Mg²⁺ containing 10⁶ CFU/ml of bacteria and 100 µl with different concentrations of F1PA in TSB supplemented with 1% glucose (Sigma-Aldrich, Merck, Darmstadt, Germany), 10 mM of Ca²⁺ and 10 mM of Mg²⁺ per well. The positive control wells contained only broth: 200 µl of TSB supplemented with 1% glucose, 10 mM of Ca²⁺ and 10 mM of Mg²⁺ containing 10⁶ CFU/mL of bacteria per well. The plate was incubated at 37°C and 5% CO₂ for at least 18 h. After incubation, the medium was removed and the wells were washed one time with sterile saline serum (0.9% NaCl). The medium was then removed, and fixation was done with 150 µl of methanol for 20 min. After fixation, methanol was removed and microtiter plates were dried at room temperature for 10 min. A volume of 200 µl of safranine (Panreac, Barcelona, Spain) was added to each well and left for 15 min at room temperature. The dye was then removed, and this was followed by 2 washings with 250 µl sterile distilled water. The dye bound to the cells were resolubilized with 200 µl of 95% ethanol per well and thereafter the microtiter plate was covered with the lid, and was dried at room temperature for at least 5 min without shaking. Biofilm formation was determined by measuring the absorbance at 492 nm.

### Example 1.12. Inhibition of wound-like medium (WLM) biofilm formation by bacteriophage

Wound-like medium (WLM) biofilm formation on the bottom of a 24-well flat-bottom plate was induced by inoculating 0.5 ml of WLM containing (50% bovine serum adult (BSA), 35% Bolton broth base, 5% laked horse red blood cells (Thermo Fisher Scientific, Waltham, MA, USA), and supplemented with 10 mM of Ca²⁺ and 10 mM of Mg²⁺) containing coagulase plasma rabbit with EDTA (Becton & Dickinson, Franklin Lakes, New Jersey, USA) and 10⁶ CFU/mL of bacteria. The plate was incubated at 37°C and 5% CO₂ for at least 18 hours. After incubation, 1 ml per well with different concentrations of F1PA in saline serum (0.9% NaCl) supplemented with 10 mM of Ca²⁺ and 10 mM of Mg²⁺ were deposited and the plate was incubated at 37°C and 5% CO₂ for 6 hours. After incubation, the content of the wells was deposited in tubes and sonicated for 5 min at room temperature. After sonication, pseudomonal bacteria concentration (CFU/cm³) was determined by drop plate cultivation method.

### Example 1.13. Statistical Analysis

Data distribution was evaluated using Shapiro-Wilk or Kolgomorov-Smirnov statistics. Descriptive statistics are cited as median and interquartile range (non-normal distribution) for each variable that were calculated. Non-parametric Mann-Whitney test considering equality of variances was used to compare two groups and non-parametric Kruskal-Wallis's test was used to compared more than two groups. To determine the effect of temperature on the phage viability, bacteriophage's adsorption and burst size, data was analysed using linear regression.

Bacteriophage inhibition of bacterial biofilm was analysed by Dunn's pairwise test with a Benjamini-Hochberg's procedure. The possible relation between the bacterial biofilm or planktonic concentration and the concentration of phage was determined by using Spearman rank correlation coefficient. Significance level was established at α=0.05.

All statistical analysis was performed using R (R Core Team, 2017) with R commander , except for linear regressions that were carried out using GraphPad Prism v.8 (GraphPad Prism, version 8.0.1 (86); Windows Version by Software MacKiev ^{©} 2020-2018 GraphPad Software, LLC.; San Diego, CA, USA) and STATA statistical software, release 11 (StataCorp, 2009, StataCorp LP., Texas, USA).

Departing from these materials and methods, and the common general knowledge, the present invention could be reproduced by the person skilled in the art departing from the genetic material of the bacteriophage (SEQ ID NO: 1). For instance, the reference [Jiang S, Tang Y, Xiang L, Zhu X, Cai Z, Li L, Chen Y, Chen P, Feng Y, Lin X, Li G, Sharif J, Dai J. Efficient de novo assembly and modification of large DNA fragments. Sci China Life Sci. 2021 Dec 16. doi: 10.1007/s11427-021-2029-0. Epub ahead of print. PMID: 34939159*]* explains how to synthesise de novo long DNA from sequences. On the other hand, the reference *[*Diana P. Pires et al., 2021. Designing P. aeruginosa synthetic phages with reduced genomes. Scientific Reports | (2021) 11:2164 | https://doi.org/10.1038/s41598-021-81580-2] explains how phages can be synthesized and a general scheme is provided therein (see Figure 2 of this paper).

### Example 2. Results

### Example 2.1. Bacteriophage Stability testing

F1PA was tested against a wide pH range (pH 1, 4.5, 7.4 and 8), over 1h incubation period, to establish their stability under acidic and alkaline conditions **(****Fig. 1****).** F1PA exhibited a slight but significant increase in viability at pH 4.5, 7.4 and 8 increasing its concentration by 2%, 3% and 4%, respectively, (p-value<0.05) at 1 h. After 1 h incubation at pH=1, no plaques were detected which suggests there is no active phage at that pH (p-value<0.05).

F1PA was stable at 4°C and did not lose viability after 1 h, 24 h and 168 h incubation **(****Fig. 2****).** At 60°C, 37°C, 21°C, -20°C and -80°C the phage titer was significant reduced only by 6% (R²=0.9099, p-value>0.0001), 0.45% (R²=0.8953, p-value>0.0001), 0.40% (R²=0.5931, p-value>0.001), 0.8% (R²=0.8659, p-value>0.0001) and 1.4% (R²=0.9071, p-value>0.0001) respect to the initial concentration, respectively.

### Example 2.2. Bacteriophage Adsorption Assays

The bacteriophage planktonic binding properties was determined through adsorption assay **(****Fig. 3****).** F1PA showed a strong adsorption to isolates being adsorbed 3% of phage titre (PFU·mL⁻¹) per minute (R²: 0.7446, p-value<0.001).

### Example 2.3. Bacteriophage one-step growth experiment

Based on the one-step growth experiment, latent period and burst size for F1PA phage was calculated **(****Fig. 4****).** The latent period was 50 min for F1PA. The burst size was approximately 96±37 particles per bacterial cell. Until 40 min of experiment there was no release of bacteriophage particles (R²= 0.0223, p-value=0.4151). However, between 40 and 80 min the release of bacteriophage particles follows a lineal tendency increasing by 30% PFU·mL⁻¹·min⁻¹ (R²= 0.9292, p-value<0.0001).

### Example 2.4. Bacteriophage Host Range Analysis

The host range against 35 *P. aeruginosa* clinical isolates for phage F1PA 22/35 susceptible (+) and 13/35 non-susceptible (-) strains, indicating strong lytic activity and a broad host range **(Table 1).** Within those 22 sensitive strains, there were eight multi-drug resistant (MDR) *P. aeruginosa* strains, (which includes PA24, PA35 and, Pa36) and four extensively-drug resistant (XDR) (which includes PA35).

### Example 2.5. Bacteriophage Inhibition assays

The F1PA planktonic infective properties were determined through inhibition assays **(****Fig. 5****).** The phage inhibition of clinical isolates was assessed for 22 h when infected at a MOI=10, MOI=1 and MOI=0.1 for PA24 **(****Fig. 5a****)** and MOI=1 and MOI=0. 1for PA35 **(****Fig. 5b****)** and PA36 **(****Fig. 5c****).**

**Table 1**

| **Strain** | **Origin** | **MDR/XDR** | **F1PA** |
|---|---|---|---|
| **PA1** | Blood | - | + |
| **PA2** | Blood | - | + |
| **PA3** | Prosthetics | - | - |
| **PA4** | Blood | - | - |
| **PA5** | Peritoneum | - | - |
| **PA6** | Ulcer | - | + |
| **PA7** | Wound | - | + |
| **PA8** | Wound | - | + |
| **PA9** | Wound | MDR | + |
| **PA10** | Ulcer | | + |
| **PA11** | Ulcer | - | + |
| **PA12** | Wound | - | + |
| **PA13** | Wound | - | - |
| **PA14** | Sputum | MDR | - |
| **PA16** | Sputum | - | + |
| **PA17** | Wound | - | + |
| **PA18** | Bronchial | XDR | + |
| **PA19** | Wound | - | + |
| **PA20** | Bronchial | MDR | - |
| **PA21** | Bronchial | MDR | - |
| **PA22** | Wound | - | + |
| **PA23** | Bronchial | XDR | + |
| **PA24** | Sputum | MDR | + |
| **PA25** | Sputum | MDR | - |
| **PA26** | Sputum | - | - |
| **PA27** | Bronchial | - | + |
| **PA28** | Sputum | XDR | - |
| **PA29** | Wound | - | + |
| **PA30** | Wound | - | + |
| **PA31** | Bronchial | - | - |
| **PA32** | Bronchial | - | - |
| **PA33** | Perianal | XDR | + |
| **PA34** | Otic | - | - |
| **PA35** | - | XDR | + |
| **PA36** | Bronchial | MDR | + |

| | | | |
|---|---|---|---|
| *Summary of F1PA Host Range. Based on the degree of lysis on the bacterial lawn, the spots were differentiated into two categories: susceptible* (+) *or non-susceptible (-)* | | | |

### Example 2.6. Minimum Inhibitory Biofilm Concentration

The concentration of PAO1 in planktonic state decreased by 63% in the presence of any concentration of bacteriophage (p-value<0.05) at 6h **(****Fig. 6a****).** The concentration of planktonic bacteria and the concentration of bacteriophages showed a strong negative correlation (p= -0.9168, p-value<0.0001) at 6h. Concentrations of 10⁹, 10⁸, 10⁷ and 10⁶ PFU·mL⁻¹ F1PA were able to reduce by 35%, 29%, 26% and 22% the amount of PAO1 biofilm, respectively, (p-value<0.05) at 6h **(****Fig. 6b****).** The amount of biofilm and the concentration of phage showed a moderate negative correlation (p= -0.5354, p-value<0.0001) at 6h. Concentrations of 10⁹, 10⁷, 10⁶ and 10⁵ PFU·mL⁻¹ of F1PA were able to reduce the concentration of PAO1 in planktonic state by 32% (p-value<0.0001) at 24 h **(****Fig. 6c****).** The concentration of planktonic bacteria and the concentration of bacteriophage showed a weak positive correlation (p= 0.2342, p-value=0.0216) at 24 h. The concentrations 10⁸, 10⁷, 10⁶ and 10⁵ PFU·mL⁻¹ of F1PA reduced the amount of PAO1 biofilm by 27% (p-value<0.001) at 24 h **(****Fig. 6d****).** There was no correlation between the amount of biofilm and the bacteriophage concentration (p-value=0.107) at 24 hours.

Concentration of PA24 clinical isolate in planktonic state increased by 30% in the presence of any concentration of bacteriophage (p-value<0.01) at 6 h **(****Fig. 7a****).** The concentration of PA24 and F1PA showed a strong positive correlation (p=0.6545, p-value<0.0001) at 6 h. Only the concentration of 10⁹ PFU·mL⁻¹ was able to reduce the amount of PA24 biofilm by 27% (p-value<0.01) at 6h **(****Fig. 7b****).** The amount of biofilm and phage showed a weak negative correlation (p= -0.3491, p-value<0.0005) at 6 h. Only the concentration of 10⁹ PFU·mL⁻¹ F1PA was able to reduce the concentration of PA24 in planktonic state by 37% (p-value=0.0003) at 24 h **(****Fig. 7c****).** There was no correlation between planktonic bacteria and bacteriophage concentration (p-value=0.2532). Concentrations 10⁹, 10⁸ and 10⁷ PFU·mL⁻¹ F1PA reduced 76%, 62% and 33% the amount of PA24 biofilm, respectively, (p-value<0.01) at 24 h **(****Fig. 7d****).** The bacteriophage concentration and the amount of biofilm showed a very strong negative correlation (p=-0.8439, p-value<0.0001) at 24 h.

Concentration of PA35 clinical isolate in planktonic state increased by 29% in the presence of any concentration of bacteriophage (p-value<0.01) at 6 h **(****Fig. 8a****).** The concentration of planktonic bacteria and bacteriophage showed a strong positive correlation (p= 0.6164, p-value<0.0001) at 6 h. Concentrations of 10⁹ and 10⁸ PFU·mL⁻¹ F1PA were able to reduce the amount ofPA35 biofilm by 83% and 23%, respectively (p-value<0.05) at 6 h **(****Fig. 8b****).** The amount of biofilm and F1PA concentration showed a moderate negative correlation (p= -0.5980, p-value<0.0001) at 6h. Concentrations of 10⁸, 10⁷, and 10⁵ PFU·mL⁻¹ of F1PA were increased by 29%, 25% and 16% the concentration of PA35 in planktonic state, respectively, (p-value<0.01) at 24 h **(****Fig. 8c****).** There was no correlation between the concentration of planktonic bacteria and F1PA concentration (p-value=0.3937) at 24 h. Concentrations 10⁹ and 10⁸ PFU·mL⁻¹ F1PA reduced the amount of PA35 biofilm by 78% and 61%, respectively, (p-value<0.0001) at 24 h **(****Fig. 8d****).** The amount of biofilm and F1PA concentration showed a strong negative correlation (p= -0.7515, p-value<0.0001) at 24 h.

Concentration of PA36 in planktonic state decreased by 21%, 25% and 29% in the presence of 10⁷, 10⁶ and 10⁵PFU ·mL⁻¹ F1PA concentrations, respectively, (p-value<0.01) at 6 h **(****Fig. 9a****).** The concentration of planktonic bacteria and the concentration of bacteriophage showed a moderate positive correlation (p= 0.4463, p-value<0.0001) at 6 h. Concentrations of 10⁹ and 10⁸ PFU·mL⁻¹ F1PA were able to reduce the amount of PA36 biofilm by 84% and 74%, respectively, (p-value<0.0001) at 6 h **(****Fig. 9b****).** The amount of biofilm and the concentration of F1PA showed a strong negative correlation (p= -0.7908, p-value<0.0001) at 6h. Only 10⁹ PFU·mL⁻¹ F1PA concentration was able to significantly reduce the concentration of PA36 in the planktonic state by 10% (p-value<0.0001) at 24 h **(****Fig. 9c****).** The concentration of planktonic bacteria and the concentration of F1PA showed a weak negative correlation (p= -0.3091, p-value=0.0022) at 24 h. Concentrations 10⁹ and 10⁸ PFU·mL⁻¹ F1PA reduced the amount of the PA36 biofilm by 68% and 20%, respectively, (p-value<0.01) at 24 h **(****Fig. 9d****).** The amount of biofilm and the concentration of F1PA showed a strong negative correlation (p= -0.6018, p-value<0.0001) at 24 h.

### Example 2.7. Effect of F1PA combined with antibiotics in biofilms

Only the combination of aztreonam, at median human plasma concentration (28.6 µg/ml), with concentrations of 10⁹ and 10⁸ PFU/ml of FlPa was able to reduce the concentration of PAO1 bacteria derived from biofilm by 80% and 79%, respectively, (p-value<0.05) at 24h in comparison with single treatment with aztreonam **(Figure 10a)**. The concentration of PAO1 and F1PA showed a very strong negative correlation (p= -0.8260, p-value<0.0001) at 24h. The concentration of 10⁹ PFU/ml F1PA combined with aztreonam was able to reduce the concentration of PA24 bacteria derived from biofilm by 88% (p-value<0.05) at 24h in comparison with a single treatment with aztreonam **(Figure 10b)**. The concentration of PA24 and F1PA showed a strong negative correlation (p=-0.6833, p-value<0.0001) at 24h.

The concentration of PAO1 bacteria derived from biofilm decreased by 75% in the presence of the combination of any concentration of bacteriophage (p-value<0.05) with piperacillin-tazobactam, at median human plasma concentration (64.3 µg/ml) ², at 24h in comparison with single treatment with piperacillin-tazobactam **(****Figure 11a****)**. The concentration of PAO1 and F1PA showed a strong negative correlation (p=-0.7934, p-value<0.0001) at 24h. Only the combination of piperacillin-tazobactam with concentration of 10⁹ PFU/ml of bacteriophage was able to reduce the concentration of PA24 bacteria derived from biofilm by 87% (p-value<0.05) at 24h in comparison with single treatment with piperacillin-tazobactam **(****Figure 11b****)**. The concentration of PA24 and F1PA showed a weak negative correlation (p= -0.3329, p-value<0.0001) at 24h.

### Example 2.8. Effect of F1PA to inhibit biofilm formation

The formation of biofilm of PAO1 was 30% lower in the presence of any concentration of bacteriophage (p-value<0.05) at 24h **(****Figure 12a****).** The formation of biofilm of PAO1 and concentration of F1PA showed a strong negative correlation (p=-0.7103, p-value<0.0001) at 24h. Concentrations of 10⁹ and 10⁸ PFU/ml F1PA were able to reduce the formation of biofilm of clinical PA24 by 83% and 92% (p-value<0.05), respectively **(****Figure 12b****).** The formation of biofilm of PA24 and concentration of F1PA showed a strong negative correlation (p=-0.7022, p-value<0.0001) at 24h. Only concentration of 10⁹ PFU/ml F1PA was able to diminish the formation of biofilm of PA35 by 90% (p-value<0.05) **(****Figure 12c****).** The formation of biofilm of PA35 and concentration of F1PA showed a moderate negative correlation (p=-0.5867, p-value<0.0001) at 24h. Concentration of 10⁹ PFU/ml F1PA was able to reduce the formation of biofilm of PA36 by 82% (p-value<0.05) **(****Figure 12d****).** The formation of biofilm of PA36 and concentration of F1PA showed a strong negative correlation (p= -0.6215, p-value<0.0001) at 24h.

### Example 2.9. Effect of F1PA in a wound-like medium

Concentration of 10⁹ PFU/ml F1PA was able to reduce the concentration of bacteria PAO1 by 2% (p-value<0.05) in the wound-like medium (WLM) at 24h **(****Figure 13****).**

## Claims

1. Bacteriophage deposited at the German Collection of Microorganisms and Cell Cultures GmbH (DSMZ) with deposit number DSM 34223.

2. Bacteriophage, according to claim 1, for use as a medicament.

3. Bacteriophage for use, according to claim 2, in the prevention or treatment of a bacterial infection caused by *P. aeruginosa.*

4. Pharmaceutical composition comprising the bacteriophage of claim 1 and, optionally, pharmaceutically acceptable excipients or carriers.

5. Pharmaceutical composition of claim 4 for use as a medicament.

6. Pharmaceutical composition for use, according to claim 5, in the prevention or treatment of a bacterial infection caused by *P. aeruginosa.*

7. A method of detecting the presence of *P. aeruginosa* in an *in vitro* sample, comprising contacting said sample with the bacteriophages according to claim 1, and determining whether said bacteriophages kill bacteria in said sample.
